# EUROPÄISCHE PATENTSCHRIFT

(11) **EP 1 555 040 B1**
(45) Veröffentlichungstag und Bekanntmachung des Hinweises auf die Patenterteilung: **26.08.2015**
(21) Anmeldenummer: 04024720.7
(22) Anmeldetag: 16.10.2004
(51) Int. Cl.: A61M 16/06, A61M 16/08, F16L 27/04

(54) **Vorrichtung zur Beatmung**
Breathing device
Dispositif respiratoire

(30) Priorität: 14.01.2004 DE 102004002125
(43) Veröffentlichungstag der Anmeldung: 20.07.2005
(73) Patentinhaber: Weinmann Geräte für Medizin GmbH + Co. KG, 22525 Hamburg (DE)
(72) Erfinder: Eifler, Martin, 25348 Glückstadt (DE); Schulz, Gerd, 22869 Schenefeld (DE)
(74) Vertreter: Patentanwälte Klickow & Partner Partnerschaftsgesellschaft mbB

(56) Entgegenhaltungen:
- EP-A- 1 334 742
- CH-A- 138 387
- DE-C- 942 427
- FR-A- 2 709 328
- US-A- 1 911 938
- US-A- 3 544 137

## Beschreibung

Die Erfindung betrifft eine Vorrichtung zur Beatmung, die als eine mit einem Atemgasschlauch verbindbare Atemmaske ausgebildet ist und die eine Schlauchkupplung für den Atemgasschlauch aufweist, die über ein Gelenk beweglich mit einem Grundkörper der Atemmaske verbunden ist, sowie bei der das Gelenk kugelgelenkartig ausgebildet ist und ein kugelsegmentartiges Innenteil sowie eine Außenschale zur Führung des Innenteiles aufweist.

Derartige Atemmasken können sowohl als Nasalmasken als auch als Vollgesichtsmasken ausgebildet sein. Typischerweise wird ein beweglicher Schlauchanschluß verwendet, der im Bereich des Grundkörpers der Maske drehbar angeordnet ist. Die gemäß dem Stand der Technik bekannten Gelenke zur Realisierung des beweglichen Schlauchanschlusses können noch nicht alle Anforderungen im vollen Umfang zufriedenstellend erfüllen, die insbesondere bei einer Anwendung durch technisch weniger begabte Patienten gestellt werden. Die Gelenke müssen zum einen eine hohe Beweglichkeit bereitstellen, um die Einleitung von Kräften über den Atemgasschlauch in die Atemmaske möglichst zu minimieren und hierdurch einen großen Benutzungskomfort zu unterstützen. Darüber hinaus müssen ein geringes Baugewicht und eine hohe Zuverlässigkeit bei einer Vielzahl von unterschiedlichen Anwendungsrandbedingungen gewährleistet werden.

Aus der EP-A-1 334 742 ist es bereits bekannt, eine Atemmaske über einen Beatmungsschlauch mit einer Atemgasversorgung zu koppeln. Beabstandet zur Atemmaske ist im Bereich des Schlauches ein Kugelgelenk angeordnet, das aus einem Innenteil und einer Außenschale besteht.

In der CH 138 387 A wird ein Kugelgelenk beschrieben, bei dem ein Innenteil von einer mehrteiligen Außenschale geführt ist. Das Kugelgelenk verbindet Rohrenden miteinander und ermöglicht eine winklige Anordnung der Rohre relativ zueinander.

In der FR-A-2 709 328 wird eine gelenkartige Verbindung von Schlauchteilen im Bereich eines Verbrennungsmotors beschrieben, wobei ein Außenteil eine Vielzahl von zungenartigen Segmenten aufweist.

Aufgabe der vorliegenden Erfindung ist es, eine Vorrichtung der einleitend genannten Art derart zu konstruieren, daß eine hohe Funktionalität bei gleichzeitig hoher Zuverlässigkeit, einfacher Montage sowie hoher mechanischer Stabilität bereitgestellt wird.

Diese Aufgabe wird erfindungsgemäß dadurch gelöst, daß die Außenschale aus mindestens zwei miteinander verbindbaren Schalensegmenten ausgebildet ist, wobei das erste Schalensegment am Grundkörper befestigt und das zweite Schalensegment ringartig ausgebildet ist.

Eine weitere erfindungsgemäße Lösung der Aufgabe besteht darin, daß das Gelenk kugelgelenkartig ausgebildet ist und ein kugelsegmentartiges Innenteil sowie eine Außenschale zur Führung des Innenteiles aufweist und daß die Außenschale aus mindestens einem Schalensegment ausgebildet ist, das in einem Montagezustand elastisch verformbare Führungselemente aufweist, die in einem Arbeitszustand von einem Fixierring stabilisiert sind.

Durch die kugelgelenkartige Ausbildung wird eine hohe Drehbeweglichkeit bei gleichzeitig großer mechanischer Stabilität bereitgestellt. Durch die Aufteilung der Außenschale in mindestens zwei miteinander verbindbare Schalensegmente ist es in einfacher weise möglich, das kugelsegmentartige Innenteil zu montieren. Gleichzeitig kann eine ausreichend große außenseitige Überdeckung des Innenteiles durch die Außenschale bereitgestellt werden, so daß ein ungewolltes Trennen der Gelenkteile voneinander ausgeschlossen ist. Insbesondere wird verhindert, daß bei einer Kraftbelastung Teile des Gelenkes in die Maske hineingedrückt werden. Dies ist insbesondere für Vollgesichtsmasken von erheblicher Bedeutung.

Eine mechanisch stabile Ausführungsform wird dadurch unterstützt, daß das erste Schalensegment am Grundkörper befestigt ist.

Zur Gewährleistung einer einfachen Montierbarkeit ist vorgesehen, daß das Innenteil als Teil der Schlauchkupplung ausgebildet ist.

Eine einfache Handhabung wird dadurch unterstützt, daß das zweite Schalensegment ringartig ausgebildet ist.

Ein Zusammenbau mit wenigen Handgriffen kann dadurch erreicht werden, daß die Schalensegmente durch mindestens eine Schnappverbindung miteinander gekoppelt sind.

Eine weitere Erhöhung der mechanischen Stabilität kann dadurch erfolgen, daß das zweite Schalensegment im Bereich eines Sicherungselementes angeordnet ist.

Eine verbesserte Führung des Innenteiles kann dadurch erreicht werden, daß das zweite Schalensegment einen das Innenteil beaufschlagenden Führungssteg aufweist.

Eine weitere Befestigungsvariante besteht darin, daß das Sicherungselement mit dem ersten Schalensegment verklemmt ist.

Ebenfalls ist es möglich, daß das Sicherungselement mit dem ersten Schalensegment verrastet ist.

Darüber hinaus ist daran gedacht, daß das Sicherungselement mit dem ersten Schalensegment verschraubt ist.

Eine hohe Drehbeweglichkeit des Gelenkes wird dadurch unterstützt, daß die Außenschale mindestens bereichsweise kugelsegmentartig ausgebildet ist.

Eine Kombination einer hohen Beweglichkeit bei gleichzeitig hoher mechanischer Stabilität kann dadurch erreicht werden, daß das erste Schalensegment eine Linienberührung zum Innenteil aufweist.

Eine einfache Geometrie der Bauteile wird dadurch unterstützt, daß das Sicherungselement als ein geschlossener Ring ausgebildet ist.

Zur Erleichterung einer Entformbarkeit von spritzgußtechnisch hergestellten Bauteilen wird vorgeschlagen, daß das Sicherungselement als ein aufklappbarer Ring ausgebildet ist.

Eine weitere Konstruktionsvariante besteht darin, daß das Sicherungselement mit einer Innenverzahnung versehen ist.

Eine örtliche Entkopplung unterschiedlicher Funktionen kann dadurch erreicht werden, daß das Gelenk außerhalb der Atemmaske zwischen dem Grundkörper und dem Atemgasschlauch angeordnet ist.

Eine gute Zugänglichkeit der eingesetzten Bauteile kann dadurch erreicht werden, daß das Sicherungselement bezogen auf einen Maskeninnenraum außenseitig angeordnet ist.

Gemäß einer gestalterisch besonders ansprechenden Ausführungsform ist vorgesehen, daß das Sicherungselement bezogen auf einen Maskeninnenraum innenseitig angeordnet ist.

Eine toleranzarme Führung des Innenteiles bei gleichzeitig einfacher Montierbarkeit sowie hoher mechanischer Stabilität wird dadurch erreicht, daß das Sicherungselement mit seiner Innenverzahnung eine Verzahnung des ersten Schalensegmentes mindestens bereichsweise umschließt.

Eine relativ große Führungsfläche für das Innenteil kann dadurch bereitgestellt werden, daß beide Schalensegmente zur Führung des Innenteiles ausgebildet sind.

Absätze durch Fertigungstoleranzen im Bereich der Führungsfläche für das Innenteil können dadurch vermieden werden, daß nur das erste Schalensegment zur Führung des Innenteiles ausgebildet ist und daß das zweite Schalensegment zur mechanischen Stabilisierung des zweiten Schalensegmentes ausgebildet ist.

In den Zeichnungen sind Ausführungsbeispiele der Erfindung schematisch dargestellt. Es zeigen:
- Fig. 1: Eine perspektivische Darstellung eines Beamtungsgerätes mit Verbindungsschlauch zu einer Beatmungsmaske,
- Fig. 2: eine perspektivische Darstellung einer Atemmaske mit Kugelgelenk,
- Fig. 3: ein Vertikalschnitt gemäß Schnittlinie III-III in Fig. 2,
- Fig. 4: eine Explosionsdarstellung der Bauteile gemäß Fig. 3,
- Fig. 5: eine gegenüber Fig. 3 abgewandelte Ausführungsform mit verkleinerter Führungsfläche im Bereich des Befestigungsringes,
- Fig. 6: eine perspektivische Darstellung eines einteiligen auseinanderklappbaren Befestigungsringes,
- Fig. 7: eine Draufsicht gemäß Blickrichtung VII auf den Befestigungsring gemäß Fig. 6,
- Fig. 8: eine weitere Abwandlung zur Ausführungsform gemäß Fig. 3, bei der der Sicherungsring innenseitig im Bereich der Atemmaske angeordnet ist,
- Fig. 9: eine Abwandlung zur Ausführungsform gemäß Fig. 8, bei der eine Verzahnung zur Bereitstellung einer Verrastung vorgesehen ist,
- Fig. 10: eine perspektivische Darstellung des Sicherungsringes gemäß Fig. 9 und
- Fig. 11: eine perspektivische Darstellung gemäß Blickrichtung XI in Fig. 9 mit abgenommenem Sicherungsring.

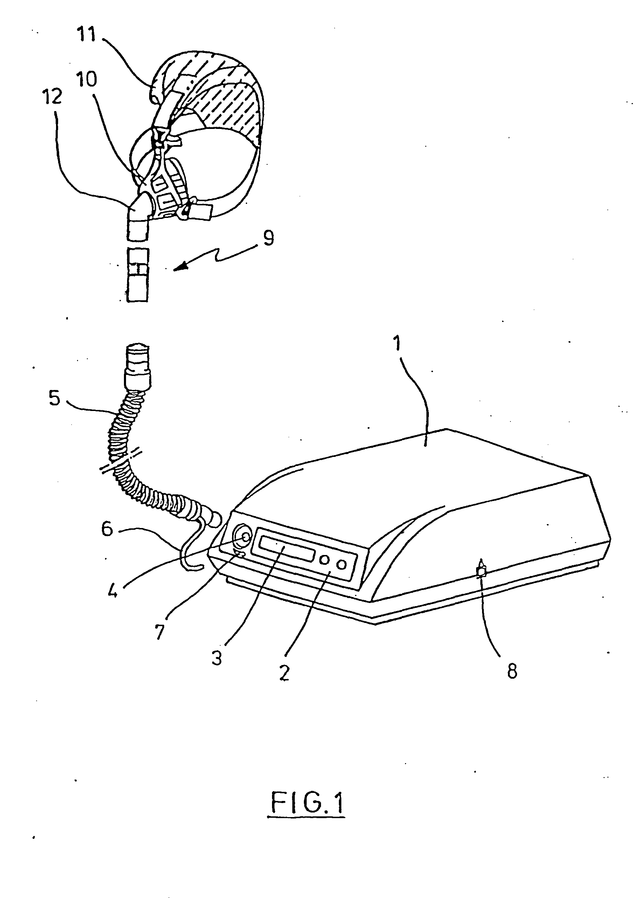

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Atemgasschlauch (5) angeschlossen. Entlang des Atemgasschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Atemgasschlauches (5) ist ein Ausatmungselement (9) angeordnet. Ebenfalls kann ein Ausatemventil verwendet werden.

Fig. 1 zeigt darüber hinaus eine Atemmaske (10), die als Nasalmaske ausgebildet ist. Eine Fixierung im Bereich eines Kopfes eines Patienten kann über eine Kopfhaube (11) erfolgen. Im Bereich ihrer dem Atemgasschlauch (5) zugewandten Ausdehnung weist die Atemmaske (10) eine Schlauchkupplung (12) auf.

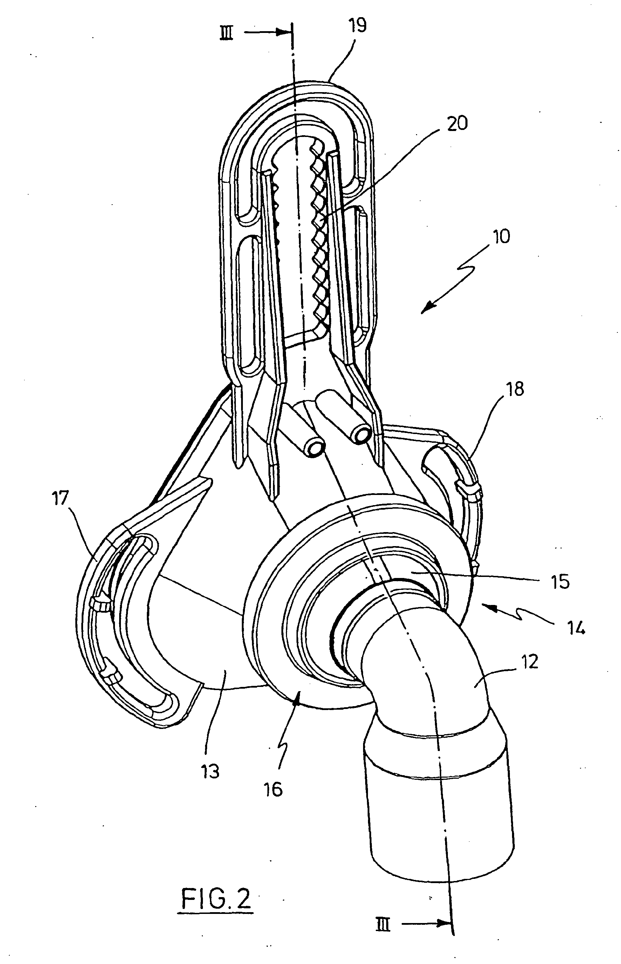

Aus Fig. 2 ist der Aufbau der Atemmaske (10) im Detail zu erkennen. Die Schlauchkupplung (12) für den nicht dargestellten Atemgasschlauch (5) ist mit einem Grundkörper (13) der Atemmaske (10) über ein Gelenk (14) verbunden, das kugelgelenkartig ausgebildet ist. Beim dargestellten Ausführungsbeispiel besteht das Gelenk (14) aus einem mit der Schlauchkupplung (12) verbundenen Innenteil (15) sowie einer mit dem Grundkörper (13) verbundenen Außenschale (16). Das Innenteil (15) ist wenigstens bereichsweise kugelsegmentartig ausgebildet und die Außenschale (16) erstreckt sich entlang wenigstens eines Teiles der kugelsegmentartigen Oberfläche des Innenteiles (15).

Am Grundkörper (13) sind seitliche Befestigungsstege (17, 18) zur Verbindung mit der in Fig. 2 nicht dargestellten Kopfhaube (11) sowie ein Mittelsteg (19) abgebildet, der ebenfalls zur Verbindung mit der Kopfhaube (11) dient und darüber hinaus eine Rastung (20) zur Positionierung einer nicht dargestellten Stirnstütze aufweist.

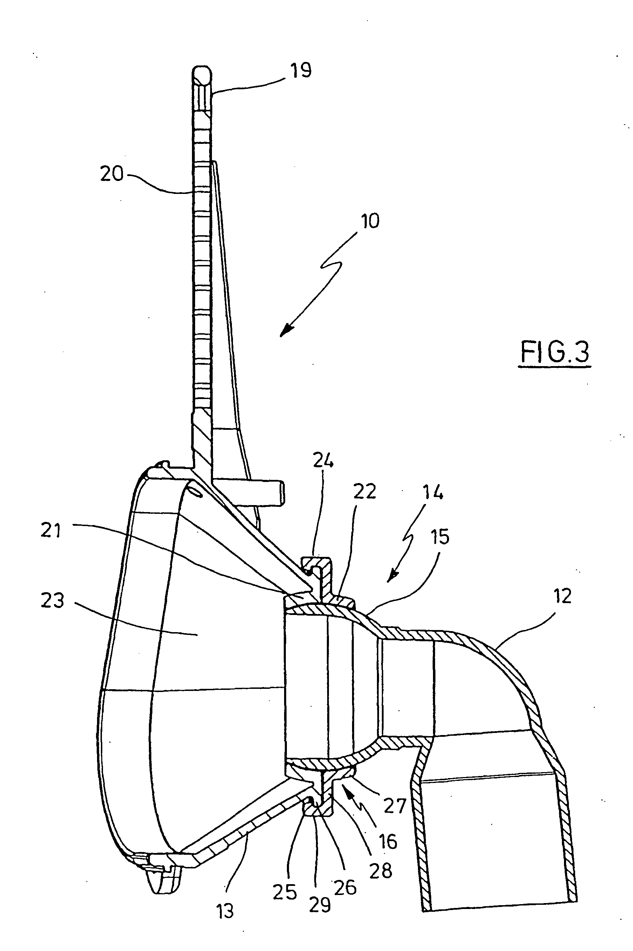

Der Vertikalschnitt in Fig. 3 veranschaulicht, daß die Außenschale (16) aus einem ersten Schalensegment (21) sowie einem zweiten Schalensegment (22) ausgebildet ist. Das erste Schalensegment (21) ist am Grundkörper (13) angeformt und erstreckt sich wenigstens bereichsweise in einen Maskeninnenraum (23) hinein. Das zweite Schalensegment (22) ist als Teil eines Sicherungselementes (24) ausgebildet, das beim dargestellten Ausführungsbeispiel als ein Sicherungsring realisiert ist.

Das Sicherungselement (24) besitzt ein Fixierprofil (25), das in ein Gegenprofil (26) des Grundkörpers (13) eingreift. Gemäß dem Ausführungsbeispiel in Fig. 3 ist das Fixierprofil (25) als ein vorstehender Rand ausgebildet, der entsprechend einer Schnappverbindung das Gegenprofil (26) hintergreift. Beim Ausführungsbeispiel gemäß Fig. 3 besteht das Sicherungselement (24) im wesentlichen aus einem am Innenteil (15) anliegenden Führungssteg (27), einem im wesentlichen senkrecht zum Führungssteg (27) verlaufenden Radialsteg (28) sowie einem Quersteg (29), der das Fixierprofil (25) trägt. Der Führungssteg (27) ist als Kugelfläche der Halbschale ausgebildet.

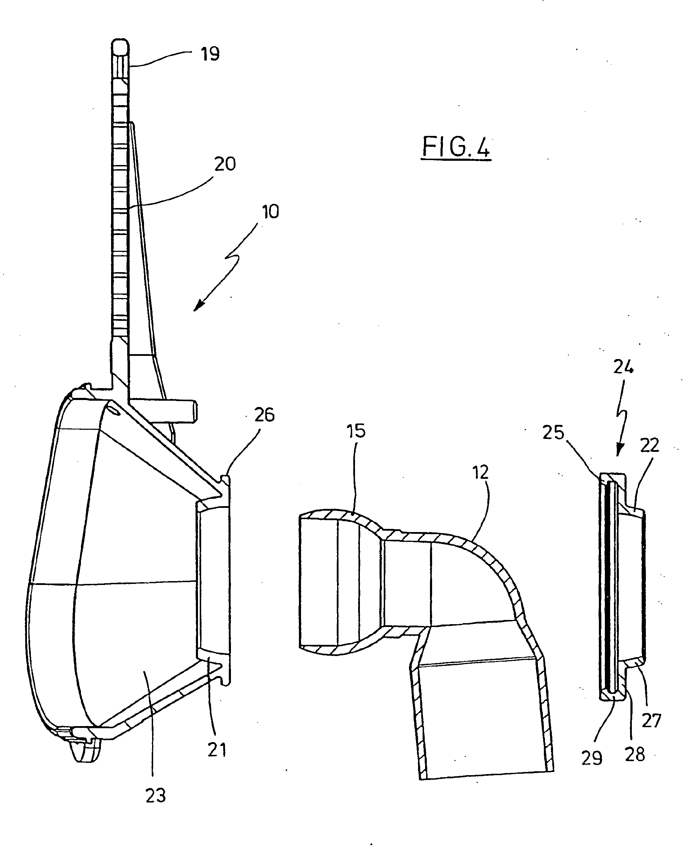

Fig. 4 veranschaulicht in einer Explosionsdarstellung die Geometrie der Einzelteile gemäß Fig. 3. Eine Montage kann beispielsweise derart erfolgen, daß zunächst das Sicherungselement (24) über die Schlauchkupplung (12) geschoben und anschließend das Innenteil (15) in das erste Schalensegment (21) hineingeschoben wird. Anschließend erfolgt ein Einrasten des Fixierprofils (25) in das Gegenprofil (26), so daß das Sicherungselement (24) am Grundkörper (13) gehalten ist. In diesem zusammengebauten Zustand ist das Innenteil (15) sowohl drehbeweglich als auch belastungsfähig von den Schalensegmenten (21, 22) geführt.

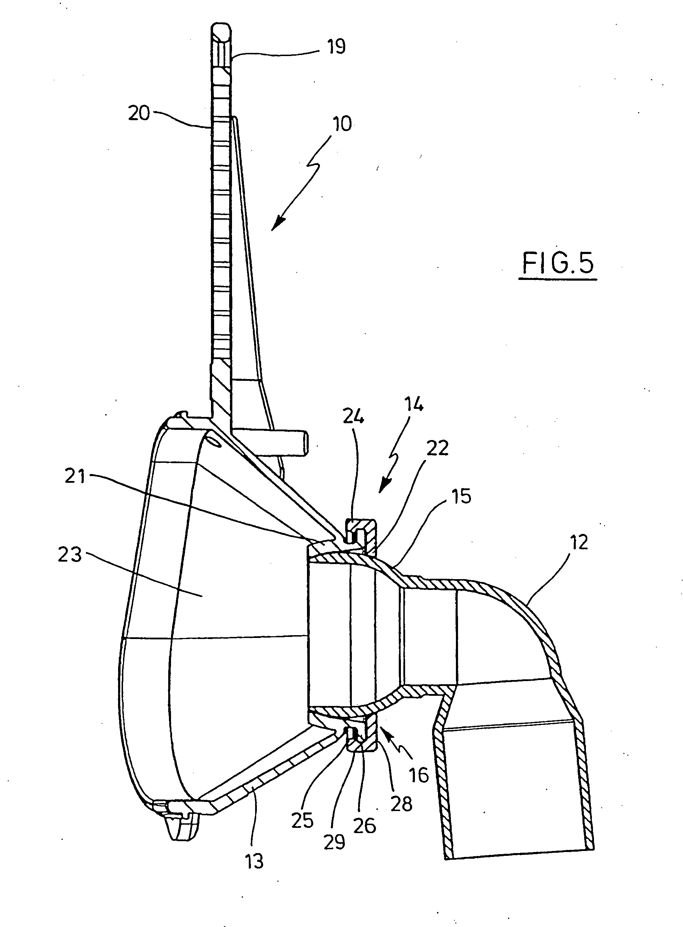

Bei der Ausführungsform gemäß Fig. 5 ist das Sicherungselement (24) ohne den Führungssteg (27) ausgebildet und das zweite Schalensegment (22) ist im Bereich der dem Innenteil (15) zugewandten inneren Begrenzung des Radialsteges (28) angeordnet. Durch diese geometrische Gestaltung wird eine einfachere Herstellung des Sicherungselementes (24) unterstützt. Das zweite Schalensegment (22) stellt eine relativ zum Kugelsegment separate Berührungsfläche bereit, die näherungsweise als eine Linienberührung realisiert ist. Die Linienberührung kann in jeder der beiden Halbschalen oder auch in beiden Halbschalen realisiert werden.

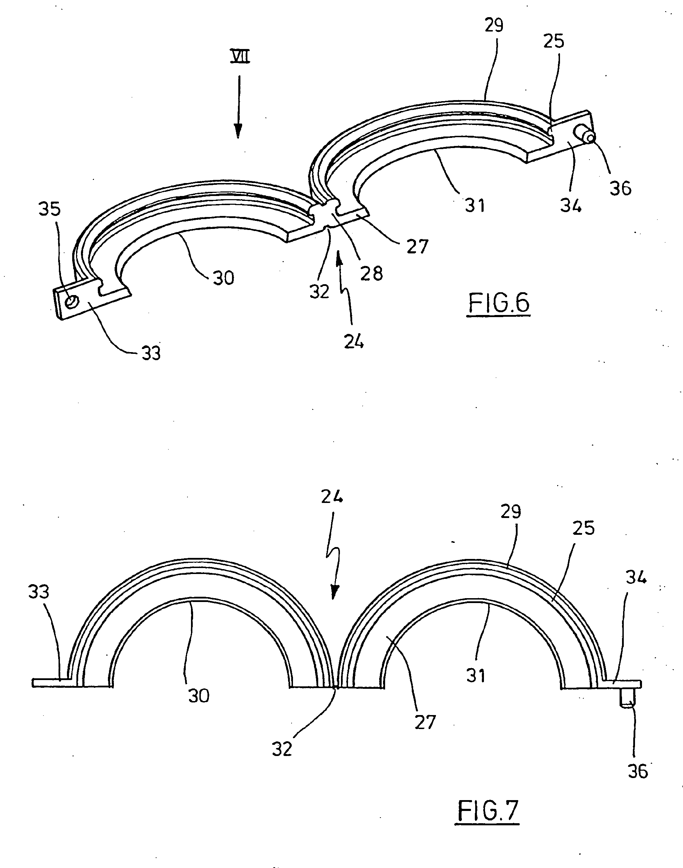

Gemäß den Darstellungen in den Figuren 6 und 7 ist das Sicherungselement (24) als ein aufklappbarer Ring ausgebildet. Das Sicherungselement (24) besteht aus zwei Ringhälften (30, 31) die über ein Filmscharnier (32) miteinander verbunden sind. Im Bereich ihrer dem Filmscharnier (32) abgewandten Begrenzungen weisen die Ringhälften (30, 31) Verbindungsstege (33, 34) auf, die derart mit einer Ausnehmung (35) sowie einem Zapfen (36) versehen sind, daß bei einem Zusammenklappen der Ringhälften (30, 31) der Zapfen (36) in die Ausnehmung (35) einrastet und hierdurch ein geschlossener Ring ausgebildet wird. Die konstruktive Realisierung des Sicherungselementes (24) ist in Fig. 6 in einer perspektivischen Darstellung und in Fig. 7 in einer Draufsicht veranschaulicht.

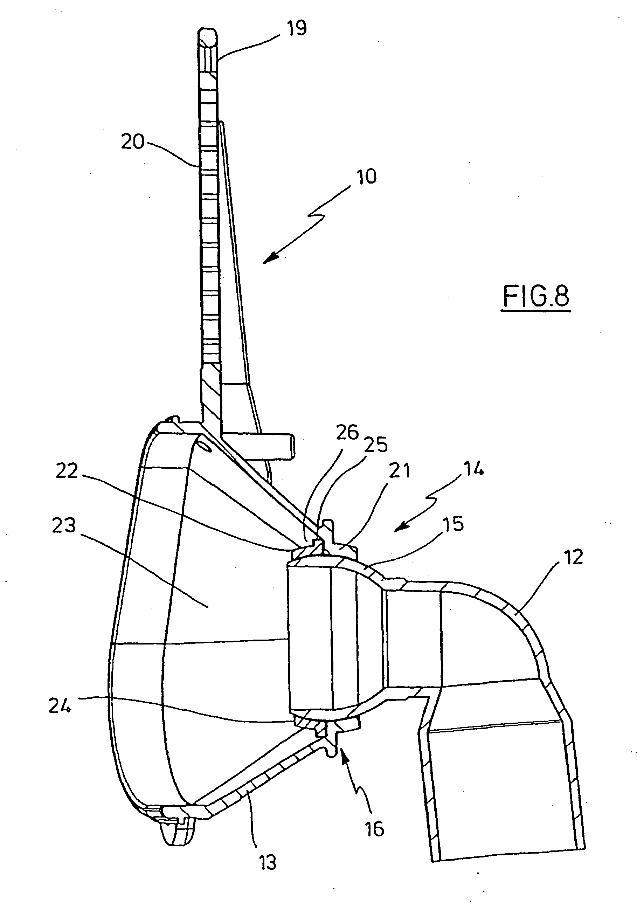

Bei der Ausführungsform gemäß Fig. 8 ist das Sicherungselement (24) mit dem zweiten Schalensegment (22) bezüglich des Maskeninnenraumes (23) innenseitig angeordnet und das mit dem Grundkörper (13) verbundene Schalensegment (21) ist im Bereich der der Schlauchkupplung (13) zugewandten Ausdehnung des Gelenkes (14) positioniert. Auch bei dieser Ausführungsform kann sich das Sicherungselement (24) im wesentlichen ringförmig erstrecken und greift mit einem Fixierprofil (25) in ein Gegenprofil (26) des Grundkörpers (13) ein.

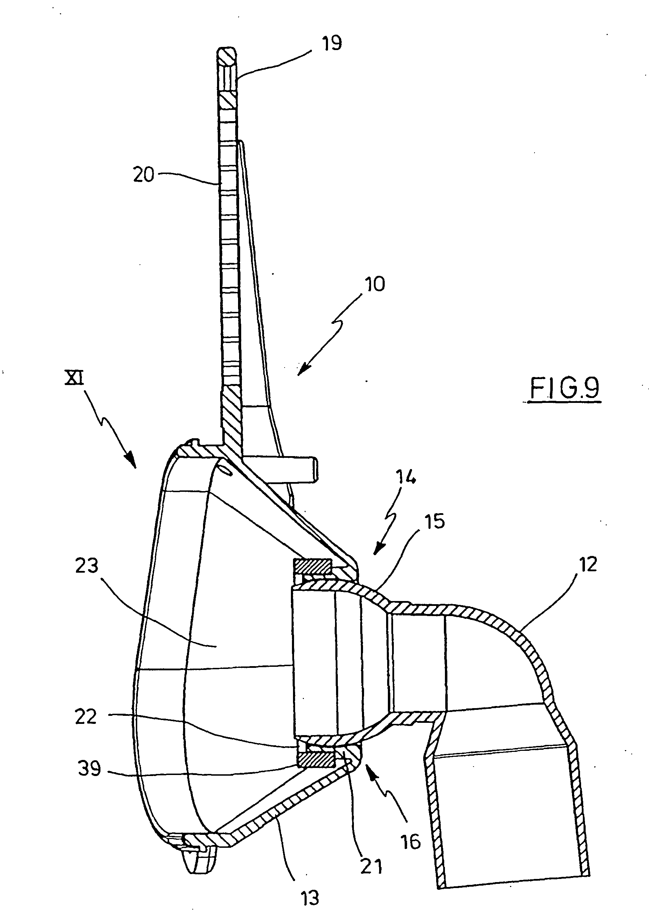

Die Ausführungsform in Fig. 9 zeigt eine zu den bisherigen Ausführungsformen grundsätzlich unterschiedliche Konstruktion. Der wesentliche Teil der Außenschale (16) wird hierbei vom ersten Schalensegment (21) ausgebildet, das am Grundkörper (13) angeformt ist. Das erste Schalensegment (21) stellt einen einteiligen Sitz für das Innenteil (15) bereit und ist in einem Montagezustand elastisch verformbar. Nach einem Aufsetzen eines Fixierringes (39) liegt keine funktionelle elastische Verformbarkeit mehr vor. Der Fixierring (39) stellt lediglich ein relativ gering dimensioniertes zweites Schalensegment (22) bereit, das im wesentlichen ein Hineindrücken des Innenteiles (15) in den Maskeninnenraum (23) hinein durch eine mechanische Stabilisierung des Lagersitzes verhindert und selbst nicht das Innenteil (15) führt.

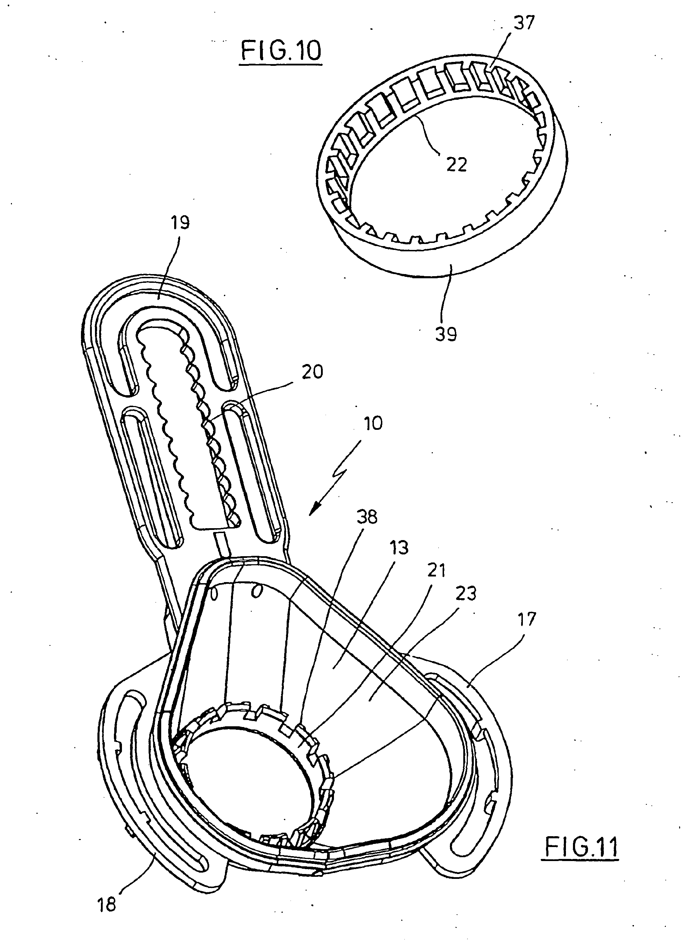

Der Aufbau des Fixierringes (39) gemäß Fig. 9 wird in Fig. 10 weiter veranschaulicht. Es ist zu erkennen, daß der Fixierring (39) im wesentlichen kreisförmig ausgebildet ist und eine Innenverzahnung (37) aufweist, die bis in den Bereich des zweiten Schalensegmentes (22) reicht. Der Fixierring (39) berührt nicht das Innenteil (15).

Aus der perspektivischen Darstellung in Fig. 11 ist zu erkennen, daß auch das erste Schalensegment (21) mit einer Verzahnung (38) versehen ist, die beim Einführen des Innenteiles (15) nach außen auseinanderdrückbar ist. Nach einer Positionierung des Innenteiles (15) sowie einem Aufschieben des Fixierringes (39) ist die Verzahnung (38) des ersten Schalensegmentes (21) gegenüber einem Aufbiegen nach außen geschützt, so daß eine sichere Halterung des Innenteiles (15) bei gleichzeitiger Gewährleistung einer ausreichenden Beweglichkeit erreicht wird.

Ein wesentlicher Vorteil der Verwendung eines einteiligen ersten Schalensegmentes (21) besteht darin, daß eine absatz- und fugenlose Führungsfläche bereitgestellt wird, so daß eine qualitativ hochwertige Leichtgängigkeit des Kugelgelenkes ermöglicht wird. Durch die in einem Montagezustand elastisch verformbaren Führungselemente ist ohne aufgesetzten Fixierring (39) eine Montage und Demontage des Innenteiles (15) mit geringem Kraftaufwand und ohne mechanische Beschädigungen der verwendeten Bauelemente möglich. Erst nach einem Aufsetzen des Fixierringes (39) wird eine im wesentlichen steife Gesamtkonstruktion bereitgestellt, die sowohl ein Herausziehen des Innenteiles (15) aus der Gelenkführung als auch ein Hineindrücken des Innenteiles in den Maskeninnenraum (13) verhindert.

## Patentansprüche

1. Vorrichtung zur Beatmung, die als eine mit einem Atemgasschlauch (5) verbindbare Atemmaske (10) ausgebildet ist und die eine Schlauchkupplung (12) für den Atemgasschlauch (5) aufweist, die über ein Gelenk (14) beweglich mit einem Grundkörper (13) der Atemmaske (10) verbunden ist, sowie bei der das Gelenk (14) kugelgelenkartig ausgebildet ist und ein kugelsegmentartiges Innenteil (15) sowie eine Außenschale (16) zur Führung des Innenteiles (15) aufweist, **dadurch gekennzeichnet, daß** die Außenschale (16) aus mindestens zwei miteinander verbindbaren Schalensegmenten (21, 22) ausgebildet ist, wobei das erste Schalensegment (21) am Grundkörper (13) befestigt und das zweite Schalensegment (22) ringartig ausgebildet ist.

2. Vorrichtung nach Anspruch 1, **dadurch gekennzeichnet, daß** das Innenteil (15) als Teil der Schlauchkupplung (12) ausgebildet ist.

3. Vorrichtung nach einem der Ansprüche 1 oder 2, **dadurch gekennzeichnet, daß** die Schalensegmente (21, 22) durch mindestens eine Schnappverbindung miteinander gekoppelt sind.

4. Vorrichtung nach einem der Ansprüche 1 bis 3, **dadurch gekennzeichnet, daß** das zweite Schalensegment (22) im Bereich eines Sicherungselementes (24) angeordnet ist.

5. Vorrichtung nach einem der Ansprüche 1 bis 4, **dadurch gekennzeichnet, daß** das zweite Schalensegment (22) einen das Innenteil (15) beaufschlagenden Führungssteg (27) aufweist.

6. Vorrichtung nach einem der Ansprüche 4 bis 5, **dadurch gekennzeichnet, daß** das Sicherungselement (24) mit dem ersten Schalensegment (21) verklemmt ist.

7. Vorrichtung nach einem der Ansprüche 4 bis 5, **dadurch gekennzeichnet, daß** das Sicherungselement (24) mit dem ersten Schalensegment (21) verrastet ist.

8. Vorrichtung nach einem der Ansprüche 4 bis 5, **dadurch gekennzeichnet, daß** das Sicherungselement (24) mit dem ersten Schalensegment (21) verschraubt ist.

9. Vorrichtung nach einem der Ansprüche 1 bis 8, **dadurch gekennzeichnet, daß** die Außenschale (17) mindestens bereichsweise kugelsegmentartig ausgebildet ist.

10. Vorrichtung nach einem der Ansprüche 1 bis 9, **dadurch gekennzeichnet, daß** das erste Schalensegment (21) eine Linienberührung zum Innenteil (15) aufweist.

11. Vorrichtung nach einem der Ansprüche 4 bis 10, **dadurch gekennzeichnet, daß** das Sicherungselement (24) als ein geschlossener Ring ausgebildet ist.

12. Vorrichtung nach einem der Ansprüche 4 bis 11, **dadurch gekennzeichnet, daß** das Sicherungselement (24) als ein aufklappbarer Ring ausgebildet ist.

13. Vorrichtung nach einem der Ansprüche 4 bis 12, **dadurch gekennzeichnet, daß** das Sicherungselement (24) mit einer Innenverzahnung (37) versehen ist.

14. Vorrichtung nach einem der Ansprüche 1 bis 13, **dadurch gekennzeichnet, daß** das Gelenk (14) außerhalb der Atemmaske (10) zwischen dem Grundkörper (13) und dem Atemgasschlauch (5) angeordnet ist.

15. Vorrichtung nach einem der Ansprüche 4 bis 14, **dadurch gekennzeichnet, daß** das Sicherungselement (24) bezogen auf einen Maskeninnenraum (23) außenseitig angeordnet ist.

16. Vorrichtung nach einem der Ansprüche 4 bis 15, **dadurch gekennzeichnet, daß** das Sicherungselement (24) bezogen auf einen Maskeninnenraum (23) innenseitig angeordnet ist.

17. Vorrichtung nach einem der Ansprüche 4 bis 16, **dadurch gekennzeichnet, daß** das Sicherungselement (24) eine verzahnung (38) des ersten Schalensegmentes (21) mindestens bereichsweise umschließt.

18. Vorrichtung nach einem der Ansprüche 1 bis 17, **dadurch gekennzeichnet, daß** beide Schalensegmente (21, 22) zur Führung des Innenteiles (15) ausgebildet sind.

19. Vorrichtung nach einem der Ansprüche 1 bis 17, **dadurch gekennzeichnet, daß** nur das erste Schalensegment (21) zur Führung des Innenteiles (15) ausgebildet ist und daß das zweite Schalensegment (22) zur mechanischen Stabilisierung des ersten Schalensegmentes (21) ausgebildet ist.

20. Vorrichtung zur Beatmung, die als eine mit einem Atemgasschlauch (5) verbindbare Atemmaske (10) ausgebildet ist und die eine Schlauchkupplung (12) für den Atemgasschlauch (5) aufweist, die über ein Gelenk (14) beweglich mit einem Grundkörper (13) der Atemmaske (10) verbunden ist, und bei der das Gelenk (14) kugelgelenkartig ausgebildet ist und ein kugelsegmentartiges Innenteil (15) sowie eine Außenschale (16) zur Führung des Innenteiles (15) aufweist, **dadurch gekennzeichnet, daß** die Außenschale (16) aus mindestens einem Schalensegment (21, 22) ausgebildet ist, das im Bereich des Grundkörpers (13) angeordnet ist und das in einem Montagezustand elastisch verformbare Führungselemente aufweist, die in einem Arbeitszustand von einem Fixierring (39) stabilisiert sind.

## Claims

1. A breathing device which is constructed as a breathing mask (10) which can be connected to a breathing-gas hose (5) and which has a hose coupling (12) for the breathing-gas hose (5), which hose coupling is movably connected by way of a joint (14) to a base body (13) of the breathing mask (10), and in which the joint (14) is constructed in the manner of a ball-and-socket joint and has a spherical segment-like inner part (15) and an outer shell (16) for guiding the inner part (15), **characterised in that** the outer shell (16) is constructed from at least two mutually connectable shell segments (21, 22), with the first shell segment (21) being fastened to the base body (13) and the second shell segment (22) being constructed in the manner of a ring.

2. A device according to Claim 1, **characterised in that** the inner part (15) is constructed as part of the hose coupling (12).

3. A device according to one of Claims 1 or 2, **characterised in that** the shell segments (21, 22) are coupled to one another by at least one snap connection.

4. A device according to one of Claims 1 to 3, **characterised in that** the second shell segment (22) is arranged in the region of a locking element (24).

5. A device according to one of Claims 1 to 4, **characterised in that** the second shell segment (22) has a guide web (27) acting on the inner part (15).

6. A device according to one of Claims 4 to 5, **characterised in that** the locking element (24) is clamped to the first shell segment (21).

7. A device according to one of Claims 4 to 5, **characterised in that** the locking element (24) is latched to the first shell segment (21).

8. A device according to one of Claims 4 to 5, **characterised in that** the locking element (24) is screwed to the first shell segment (21).

9. A device according to one of Claims 1 to 8, **characterised in that** the outer shell (17) is constructed in the manner of a spherical segment, at least in certain areas.

10. A device according to one of Claims 1 to 9, **characterised in that** the first shell segment (21) is in linear contact with the inner part (15).

11. A device according to one of Claims 4 to 10, **characterised in that** the locking element (24) is constructed as a closed ring.

12. A device according to one of Claims 4 to 11, **characterised in that** the locking element (24) is constructed as a hinged ring.

13. A device according to one of Claims 4 to 12, **characterised in that** the locking element (24) is provided with an internal toothed portion (37).

14. A device according to one of Claims 1 to 13, **characterised in that** the joint (14) is arranged outside the breathing mask (10), between the base body (13) and the breathing-gas hose (5).

15. A device according to one of Claims 4 to 14, **characterised in that** the locking element (24) is arranged on the outside in relation to a mask interior (23).

16. A device according to one of Claims 4 to 15, **characterised in that** the locking element (24) is arranged on the inside in relation to a mask interior (23).

17. A device according to one of Claims 4 to 16, **characterised in that** the locking element (24) surrounds a toothed portion (38) of the first shell segment (21), at least in certain areas.

18. A device according to one of Claims 1 to 17, **characterised in that** both shell segments (21, 22) are constructed to guide the inner part (15).

19. A device according to one of Claims 1 to 17, **characterised in that** only the first shell segment (21) is constructed to guide the inner part (15) and **in that** the second shell segment (22) is constructed to mechanically stabilise the first shell segment (21).

20. A breathing device, which is constructed as a breathing mask (10) which can be connected to a breathing-gas hose (5) and which has a hose coupling (12) for the breathing-gas hose (5), which hose coupling is movably connected by way of a joint (14) to a base body (13) of the breathing mask (10), and in which the joint (14) is
constructed in the manner of a ball-and-socket joint and has a spherical segment-like inner part (15) and an outer shell (16) for guiding the inner part (15), **characterised in that** the outer shell (16) is constructed from at least one shell segment (21, 22) which is arranged in the region of the base body (13) and which, in an assembled state, has resiliently deformable guide elements which are stabilised by a fixing ring (39) in an operating state.

## Revendications

1. Dispositif de respiration qui est configuré en tant que masque respiratoire (10) pouvant être relié à un tuyau de gaz respiratoire (5) et qui est doté d'un dispositif de couplage de tuyau (12) qui, destiné au tuyau de gaz respiratoire (5), est relié de manière mobile à un corps de base (13) du masque respiratoire (10) par l'intermédiaire d'une articulation (14), sachant que l'articulation (14) est configurée dans le genre d'une articulation à rotule et est dotée d'une pièce intérieure (15) genre segment sphérique, ainsi que d'une coque extérieure (16) pour le guidage de la pièce intérieure (15), **caractérisé en ce que** la coque extérieure (16) est composée d'au moins deux segments de coque (21, 22) qui peuvent être reliés ensemble, sachant que le premier segment de coque (21) est fixé au corps de base (13) et que le deuxième segment de coque (22) est configuré en forme de bague.

2. Dispositif selon la revendication 1,
**caractérisé en ce que** la pièce intérieure (15) est réalisée sous la forme d'un composant d'un dispositif de couplage de tuyau (12).

3. Dispositif selon l'une des revendications 1 ou 2,
**caractérisé en ce que** les segments de coque (21, 22) sont couplés ensemble au moyen d'au moins un encliquetage.

4. Dispositif selon l'une des revendications 1 à 3,
**caractérisé en ce que** le deuxième segment de coque (22) est disposé dans la région d'un élément de sécurité (24).

5. Dispositif selon l'une des revendications 1 à 4,
**caractérisé en ce que** le deuxième segment de coque (22) est doté d'une patte de guidage (27) qui agit sur la pièce intérieure (15).

6. Dispositif selon l'une des revendications 4 à 5,
**caractérisé en ce que** l'élément de sécurité (24) est coincé avec le premier segment de coque (21).

7. Dispositif selon l'une des revendications 4 à 5,
**caractérisé en ce que** l'élément de sécurité (24) est enclenché avec le premier segment de coque (21).

8. Dispositif selon l'une des revendications 4 à 5,
**caractérisé en ce que** l'élément de sécurité (24) est vissé avec le premier segment de coque (21).

9. Dispositif selon l'une des revendications 1 à 8,
**caractérisé en ce que** la coque extérieure (17) est réalisée, au moins par sections, à la manière d'un segment de sphère.

10. Dispositif selon l'une des revendications 1 à 9,
**caractérisé en ce que** le premier segment de coque (21) est doté d'un contact par lignes vers le pièce intérieure (15).

11. Dispositif selon l'une des revendications 4 à 10,
**caractérisé en ce que** l'élément de sécurité (24) est réalisé sous la forme d'un anneau fermé.

12. Dispositif selon l'une des revendications 4 à 11,
**caractérisé en ce que** l'élément de sécurité (24) est réalisé sous la forme d'un anneau pliable.

13. Dispositif selon l'une des revendications 4 à 12,
**caractérisé en ce que** l'élément de sécurité (24) est doté d'une denture intérieure (37).

14. Dispositif selon l'une des revendications 1 à 13,
**caractérisé en ce que** l'articulation (14) est disposée en dehors du masque respiratoire (10), entre le corps de base (13) et le tuyau de gaz respiratoire (5).

15. Dispositif selon l'une des revendications 4 à 14,
**caractérisé en ce que** l'élément de sécurité (24) est disposé du côté extérieur par rapport à un espace intérieur du masque (23).

16. Dispositif selon l'une des revendications 4 à 15,
**caractérisé en ce que** l'élément de sécurité (24) est disposé côté intérieur par rapport à un espace intérieur du masque (23).

17. Dispositif selon l'une des revendications 4 à 16,
**caractérisé en ce que** l'élément de sécurité (24) entoure, au moins par sections, une denture (38) du premier segment de coque (21).

18. Dispositif selon l'une des revendications 1 à 17,
**caractérisé en ce que** les deux segments de coque (21, 22) sont configurés pour le guidage de la pièce intérieure (15).

19. Dispositif selon l'une des revendications 1 à 17,
**caractérisé en ce que** seul le premier segment de coque (21) est configuré pour le guidage de la pièce intérieure (15) et que le deuxième segment de coque (22) est configuré pour la stabilisation mécanique du premier segment de coque (21).

20. Dispositif de respiration qui est configuré en tant que masque respiratoire (10) pouvant être relié à un tuyau de gaz respiratoire (5) et qui est doté d'un dispositif de couplage de tuyau (12) qui, destiné au tuyau de gaz respiratoire (5), est relié de manière mobile à un corps de base (13) du masque respiratoire (10) par l'intermédiaire d'une articulation (14), sachant que l'articulation (14) est configurée à la manière d'une articulation à rotule et est dotée d'une pièce intérieure (15) genre segment sphérique, ainsi que d'une coque extérieure (16) pour le guidage de la pièce intérieure (15), **caractérisé en ce que** la coque extérieure (16) comprend au moins un segment de coque (21, 22) qui est disposé dans la région du corps de base (13) et qui, à un état de montage, est doté d'éléments de guidage élastiquement déformables, qui, dans un état de fonctionnement, sont stabilisés au moyen d'une bague de fixation (39).
